# EUROPEAN PATENT APPLICATION

(11) **EP 3 556 839 A1**
(43) Date of publication of application: **23.10.2019**
(21) Application number: 18168257.6
(22) Date of filing: 19.04.2018
(51) Int. Cl.: C12M 1/00

(54) **CELL SEPARATION CHAMBER AND CELL SEPARATION SYSTEM AND METHOD**

(71) Applicant: Aglaris Ltd, Stevenage, Hertfordshire SG1 2FX (GB)
(72) Inventor: HORNA TOMÁS, David, Stevenage, Hertfordshire SG1 2FX (GB); COSTA FERRANDO, Miquel, Stevenage, Hertfordshire SG1 2FX (GB)
(74) Representative: ABG Intellectual Property Law, S.L.

(57) **Abstract**

The present invention is comprised among apparatus and/or devices for cell separation. The invention relates to a cell separation chamber, a system and a method for the cell separation and cell recovery of cell systems suspended in a culture medium. In particular, the invention seeks to completely separate cell systems from their original culture medium and provides a separation with high viability for reusing the recovered cell systems and the separated culture medium.

## Description

### Object of the Invention

The present invention is comprised among apparatus and/or devices for cell separation. The invention relates to a cell separation chamber, a system and a method for the cell separation and cell recovery of cell systems suspended in a culture medium. In particular, the invention seeks to completely separate cell systems from their original culture medium and provides a separation with high viability for reusing the recovered cell systems and the separated culture medium.

### Background of the Invention

The continuous production of specific cell products is of interest, among others, in the chemical or pharmaceutical industries, for which a large number of bioreactors are needed. The focus of said industries and bioreactor manufacturers lies mainly on culturing and obtaining a cell product secreted by the cells present in a culture medium in which they are suspended inside said bioreactor.

Cell separation systems known today were designed for recovering the product secreted by cells. Nevertheless, for these known systems cell care is not important and therefore cell viability and efficiency following recovery is very low. In other words, the solutions offered today in the state of the art in terms of cell care, so that the cell can possibly be used again in the future after it has been recovered and the product of interest harvested, are not satisfactory.

There are various solutions in the state of the art for filtering and separating cells from the culture medium in which they are present.

Conventionally, filters or membranes with micropores were exclusively used, but these filters or membranes present the problem of a cake gradually forming on the filtration surface in the vicinity of the pores due to substance agglutination, which cake finally ends up blocking the pores, and therefore reducing the efficiency of the filtration system to zero. To prevent this agglutination on the pores of the filters, a possible solution known in the state of the art is to incorporate hydrophilic microparticles, such microparticles adhere to the cells, but treatment with enzymatic solutions which slows down and may even undermine the recovery of said cells is required for subsequent separation.

An example of known filtration is the tangential filtration system. This system seeks to reduce the problem of material accumulation on the filtration surface that is generated. To that end, the fluid medium is stirred by means of a stream parallel (tangent) to the filtration surface which keeps the fluid in motion and prevents substance deposition due to the continuous sweeping caused by the stream passing over the surface.

Although this tangential filtration method reduces the problems related to porous medium saturation, it has, however, a series of drawbacks when the goal is to completely separate cells in suspension from a culture medium in which they are found.

Some of these drawbacks of these tangential filtration methods in cell separation are:
- long filtration times;
- the cell systems must always be in a specific medium and in motion, making it impossible to completely separate these cell systems from the culture medium;
- the need for a large investment in both initial costs and maintenance costs due to the complexity and precision requirements in equipment design, installation and care;
- degradation of filters and/or membranes reducing their mean service life;
- reduced efficiency depending on the viscosity of the medium to be filtered;
- complex pore architecture as it is necessary to take into account the movement of the microparticles or cell systems suspended in the stirred medium with respect to the pores to favor the passage thereof through said pores and/or prevent hitting against them.

### Description of the Invention

The present invention proposes a solution to the preceding problems by means of a cell separation chamber according to claim 1, a cell separation system according to claim 7, and a cell separation method according to claim 14. The dependent claims define the preferred embodiments of the invention.

A first inventive aspect provides a cell separation chamber suitable for storing fluids and/or cell systems therein, the cell separation chamber being characterized in that it comprises:
a first fluid inlet-outlet,
a second fluid inlet-outlet, and
filtering means comprising:
   a first filter arranged in the first fluid inlet-outlet,
   a second filter arranged in the second fluid inlet-outlet, and
   a plurality of microparticles arranged in the interior of the cell separation chamber,
   wherein
the first filter comprises a first plurality of pores, each pore comprising a first pore size which is smaller than the size of each component of the cell systems and smaller than the size of the microparticles,
the second filter comprises a second plurality of pores, each pore comprising a second pore size which is smaller than the size of the microparticles and larger than the size of each component of the cell systems, and
   the cell separation chamber is configured for being in fluid communication with a duct network of a cell separation system.

Throughout this document, "fluids" will be understood as liquid media; gaseous media, such as air, for example; and cell systems suspended in liquid media. Several examples of liquid media such as culture medium, cleaning liquid and recovery medium are identified in the present invention.

In the present invention, "cell systems" will be understood to be systems made up of cells, such as eukaryotic cells and prokaryotic cells, for example; or bacteria, for example; yeasts; algae or fungi. In other words, each cell system will be formed by a plurality of components that are understood as cells, bacteria, yeasts, algae and fungi.

The fluid inlets-outlets comprised in the cell separation chamber are understood as openings or accesses to said cell separation chamber through which fluids can be introduced and extracted, i.e., accesses to the cell separation chamber which can be fluid inlets or outlets, or both. In particular, the first fluid inlet-outlet is closed by a first filter, and the second fluid inlet-outlet is in turn closed by a second filter, the first filter being characteristically different from the second filter.

Advantageously, the cell separation chamber defined in the present invention allows carrying out complete cell separation as a result of the synergy between the first filter and the plurality of microparticles arranged in the interior of the cell separation chamber. When the culture medium is passed from the interior of the cell separation chamber to the first filter of the first outlet-inlet for the separation thereof in a first flow direction, said microparticles, arranged one on top of the other, form a three-dimensional network forming a plurality of interstices where the components of the cell system will gradually accumulate, thereby preventing precipitation thereof on the first filter, blocking the pores.

Also advantageously, the present cell separation chamber allows recovering the cell system after its separation from the culture medium. Since the recovery medium is driven in a second flow direction using means for driving fluids, from a recovery medium reservoir into the cell separation chamber, the recovery medium breaks up the network of microparticles by entrainment and leaves the cell system in suspension, entraining the components of said cell system to the second inlet-outlet, passing through the second filter which, as a result of its diameter, allows the passage of each component of the cell system suspended in the recovery medium but not the microparticles.

In other words, the present cell separation chamber advantageously allows completely separating the cultured cell system from the culture medium, and in turn recovering said cell system that has already been completely separated from the culture medium. To enable achieving a complete, as well as efficient, separation of the cell system from the culture medium, the cell separation chamber has particular filtering means.

On one hand, the presence of microparticles in the interior of the cell separation chamber causes said microparticles to form a three-dimensional filter which advantageously increases the retention surface area, does not damage the cell system and allows the fluid medium to filter through this three-dimensional filter. Furthermore, the formation of this three-dimensional microparticle filter retaining the components of the cell system advantageously prevents said components of the cell system from lumping together or clustering, causing the first filter to collapse when the liquid medium is filtered therethrough. The filtration of the liquid medium is thereby favored given that the cell system is prevented from blocking the pores of the first filter.

Furthermore, the cell separation chamber has two filters, a first filter and a second filter suitable for filtering a liquid medium and a cell system suspended in the liquid medium, respectively. The first filter is a porous filter comprising a first plurality of pores, each pore having a first pore size which is smaller than the size of the components of the cell system and smaller than the size of the microparticles. The first filter therefore only allows the passage of liquid medium therethrough. Advantageously, this first filter, together with the three-dimensional filter formed by the microparticles with the cell system, allows complete separation of the culture medium of the cultured cell system. Furthermore, this first filter in collaboration with the three-dimensional filter formed by the microparticles filters the liquid medium (culture medium and/or cleaning liquid or recovery medium) when this liquid medium has a first flow direction. In other words, the system is configured for changing the flow direction of the fluids by means of the actuation of means for changing flow direction; this causes the liquid medium contained in the interior of the cell separation chamber to be extracted through the first fluid inlet-outlet, being filtered through said filter, in a first flow direction. The size of the pore opening of the first filter will be smaller than the volume of each component of the cell system such that it prevents these components from going through said pore opening; and the size of the pore opening of the second filter will be larger than the volume of each component of the cell system such that it allows these components to go through said pore opening.

The second filter is a porous filter comprising a second plurality of pores, wherein each pore has a second pore size which is smaller than the size of the microparticles and larger than the size of the components of the cell system. The second filter therefore only allows the passage of a liquid medium with a cell system suspended therein. Advantageously, this second filter allows the cell system suspended in a liquid recovery medium to be completely filtered and separated from the microparticles such that the cultured cell system is recovered completely free of culture medium. In particular, this recovered cell system is extracted through the second fluid inlet-outlet from the interior of the cell separation chamber suspended in a liquid recovery medium. In other words, the actuation of the means for changing flow direction causes the cell system and the liquid medium contained in the cell separation chamber to be extracted, free of microparticles, through the second fluid inlet-outlet, being filtered through the second filter in a second flow direction.

For example, if the cell system is made up of cells, the first filter comprises a plurality of pores the pore size of which is smaller than the size of the cells, whereas the second filter comprises a plurality of pores the pore size of which is larger than the size of the cells (though smaller than the size of the microparticles), so cell separation is achieved, i.e., cells are separated from the culture medium where they were cultured.

The cell separation chamber is configured for changing the flow direction of the content therein as a result of the actuation of means for changing flow direction arranged in a cell separation system. In other words, in the cell separation system, the cell separation chamber is in connection between other components, with means for driving fluids that are in charge of driving fluids entering and leaving the chamber, as well as means for changing flow direction. Advantageously, the combination of the filters arranged in the inlets-outlets of the cell separation chamber, the microparticles contained in the interior of this cell separation chamber, and the change in flow direction allow the cell system to be completely separated from the culture medium, and in turn recovered according to the interest of the user.

In a particular embodiment, the microparticles occupy a volume between 10% and 50% of the interior of the cell separation chamber.

Advantageously, the volume of the microparticles will be adapted to the density of the cell system in the culture medium for cell separation and recovery. In other words, the volume will depend on the effective surface area resulting from the network formed by the microparticles, arranged one on top of the other, in the fluid medium that is required for retaining a large part of the components of the cell system present in the interstices of the network.

In a particular embodiment, the microparticles are formed by polymeric materials, preferably biocompatible polymeric materials, such as polystyrene, polypropylene, polylactic acid, collagen, cellulose, chitosan or any combination thereof.

In a particular embodiment, the first filter and second filter are formed by polymeric materials, preferably biocompatible polymeric materials, such as polycarbonate, or cellulose, or nylon, or PES, or PE, or PEEK, or PET, or any combination thereof.

Advantageously, a combination of any of the materials can be selected for both microparticles and filters, according to both the requirements of the process and the nature of the fluid media and cell systems involved therein, said combination being adapted to and optimizing said cell separation and recovery process. More specifically and also advantageously, the surface of said microparticles and filters may be conferred with hydrophilic and/or hydrophobic properties.

In a particular embodiment, the first filter and second filter of the cell separation chamber has a pore size between 0.2 µm and 200 µm. More particularly, the first filter and second filter have a circular shape and a diameter size between 9 mm and 10 cm. Furthermore, the first filter and second filter have a thickness between 0.05 mm and 5 cm.

In a particular embodiment, the cell separation chamber comprises a third fluid inlet-outlet, said third fluid inlet-outlet being configured so that the fluid is preferably air. More particularly, the cell separation chamber is additionally configured for receiving and releasing pressurized air therein as a result of it being able to be in fluid communication with air injection and/or release means arranged in a cell separation system. The injection of pressurized air into and/or the release thereof into the cell separation chamber advantageously contributes to the extraction of fluids from and the introduction thereof into said cell separation chamber, such that it gives the cell separation chamber the particularity of having a high fluid circulation efficiency, i.e., filtration of these fluids, thereby achieving the complete separation of the cultured cell system from the culture medium.

Advantageously, the device may evacuate the excess pressure that is generated by introducing a fluid into the closed cell separation chamber.

In a particular embodiment, the cell separation chamber comprises a fourth fluid inlet-outlet configured for being in fluid communication with a duct network of the cell separation system.

A second inventive aspect provides a cell separation system comprising:
at least one cell separation chamber according to the first inventive aspect,
a duct network connected with the interior of the at least one cell separation chamber,
flow control means for controlling the flow of fluid circulating through the duct network,
means for driving fluids entering and leaving the at least one cell separation chamber,
at least one product receptacle connected to the duct network and suitable for housing therein fluids coming from the interior of the cell separation chamber, and
a recovery medium receptacle connected to the duct network and storing a recovery medium therein,
wherein the system is configured for changing the flow direction of the fluid entering and/or leaving the at least one cell separation chamber using means for changing flow direction.

The duct network is configured for being in fluid communication with the interior of the cell separation chamber, nevertheless, it is understood that the different receptacles comprised in the present system are configured for coming into fluid communication with the interior of the cell separation chamber through the duct network due to their individual connection to said duct network.

The duct network is formed by a plurality of ducts and/or tubes in fluid communication or in connection with one another.

The recovery medium receptacle is suitable for storing therein a liquid recovery medium that will be introduced into the interior of the cell separation chamber once all the culture medium has been filtered out of this cell separation chamber. The recovery medium is therefore introduced into the interior of the cell separation chamber for recovering the cell system which is the main objective of the operator, separating the cell system from the culture medium and recovering it in a new medium. Furthermore, this recovery medium can be used like a cleaning liquid to wash the interior of the cell separation chamber in order to remove traces of culture medium. In other words, it is understood that the recovery medium can be a medium in which the cell system may be suspended, or a culture medium that is the same as or different from the medium in which the cell system was cultured, or a cryopreservation medium, or a cleaning liquid.

The means for driving fluids are configured for pumping the fluid of interest in the flow direction allowed by the system.

In a particular embodiment, the means for driving fluids are a peristaltic pump. In another particular embodiment, the means for driving fluids are communicating vessels arranged in the duct network.

The present cell separation system comprises means for changing flow direction of the fluid entering or leaving the cell separation chamber.

In a particular embodiment, the means for driving fluids and changing the flow direction of said fluids are the same, and they are an alternating peristaltic flow pump, for example. In another particular embodiment, the means for driving fluids and changing flow direction are two pumps positioned in parallel, each in charge of pumping the fluid in a direction opposite the other one, i.e., a first pump in charge of pumping the fluid in a first flow direction, and a second pump in charge of pumping the fluid in a second flow direction.

In a particular embodiment, the means for changing flow direction of the fluids entering and leaving the cell separation chamber are means configured for rotating the system until achieving the flow direction of interest, i.e., for changing a first flow direction to a second flow direction and vice versa, for example, rotating the cell separation system 180° with respect to an initial position.

The present cell separation system has the following main advantages with respect to the state of the art:
- High cell separation efficiency. The present system prevents the filtering means from getting blocked, so it does not have a downward performance curve, rather said curve remains constant, i.e., the filtration efficiency of the present system is not affected or reduced due to filter pore saturation. The present system is also faster than other known means, such as tangential filtration, since filtration in the present system is performed in a direction essentially perpendicular to the filtration surface, i.e., the movement of the fluid entraining the cells is in a direction parallel to the direction of passage through the filter pore.
- High cell separation, i.e., complete separation of the cultured cell system from the culture medium.
- High cell viability. In other words, in the present invention, the cell system involved in the separation and recovery process does not become damaged and is suitable to be subsequently reused.
- Possibility of adding any additional culture medium. As a result of having performed a complete separation and washing of the cell separation chamber and the elements contained therein (filters, microparticles, cells), removing possible traces of any culture medium previously present in the cell separation chamber, the present invention allows adding different additional culture media or cryopreservation media through the inlet-outlet of the cell separation chamber.
- Filter architecture and/or structure. In other words, due to the relative movement of the fluid to be filtered with respect to the pores, and to the network of microparticles working together as filtering means, it is not necessary to incorporate in the present invention constructive solutions in the pores for adapting to tangential fluid movements, nor is it necessary to prevent substance agglomeration in the vicinity thereof. The high degradation that may be caused by tangential entrainment processes in the filters or membranes will not have to be taken into account either.

In a particular embodiment, the cell separation system comprises:
a residual product receptacle connected to the duct network and suitable for housing therein residual fluids coming from the interior of the cell separation chamber, and/or
an end product receptacle connected to the duct network and configured for housing therein fluids coming from the interior of the cell separation chamber, preferably cell systems suspended in a recovery medium.

The residual product receptacle advantageously allows storing therein fluids that, coming from the interior of the cell separation, circulate through the duct network chamber and are negligible. More particularly, the residue receptacle is suitable for receiving the culture medium that is free of cells and the cleaning liquid coming from the interior of the cell separation chamber.

The end product receptacle is configured for storing therein the components of the cell system that has already been completely separated from the culture medium together with a recovery medium in which said cell system is suspended.

In a particular embodiment, the system further comprises a cleaning receptacle connected to the duct network and storing a cleaning liquid therein, this cleaning receptacle being configured for releasing cleaning liquid into the interior of the cell separation chamber.

The cleaning receptacle is configured for storing a cleaning liquid therein and releasing it into the interior of the cell separation chamber. Advantageously, this cleaning liquid, for example PBS, is in charge of removing and filtering any leftover culture medium that may remain in the interior of the cell separation chamber during cell separation. Nevertheless, the cleaning liquid together with the filtering means of the cell separation chamber achieve the complete separation of the cultured cells from the culture medium.

In a particular embodiment, the system further comprises a cell separation prechamber connected to the duct network, the cell separation prechamber being suitable for storing cells suspended in a culture medium. Advantageously, the system allows the cultured cell system suspended in the culture medium, coming from a cell culturing process, to be contained in a cell separation prechamber for subsequent introduction into the cell separation chamber for separation thereof when desired by the user. Nevertheless, the cell system suspended in the culture medium is introduced into the interior of the cell separation chamber through the duct network.

In a more particular embodiment, the cell separation prechamber is in connection with the interior of the cell separation chamber through the second fluid inlet-outlet. In other words, this cell separation prechamber is configured for being in fluid communication with the interior of the cell separation chamber through the second fluid inlet-outlet by means of the duct network.

In another particular embodiment, the cell separation prechamber is in connection with the interior of the cell separation chamber through a fourth fluid inlet-outlet. In other words, this cell separation prechamber is configured for being in fluid communication with the interior of the cell separation chamber through the fourth fluid inlet-outlet by means of the duct network.

In a particular embodiment, the cleaning receptacle is in connection with the interior of the cell separation chamber through the second fluid inlet-outlet.

In another particular embodiment, the cleaning receptacle is in connection with the interior of the cell separation chamber through the fourth fluid inlet-outlet.

In a particular embodiment, the residual product receptacle is in connection with the interior of the cell separation chamber through the first fluid inlet-outlet.

In a particular embodiment, the recovery medium receptacle is in connection with the interior of the cell separation chamber through the first fluid inlet-outlet.

In another particular embodiment, the recovery medium receptacle is in connection with the interior of the cell separation chamber through the second fluid inlet-outlet.

In another particular embodiment, the recovery medium receptacle is in connection with the interior of the cell separation chamber through the fourth fluid inlet-outlet.

In a particular embodiment, the end product receptacle is in connection with the interior of the cell separation chamber through the second fluid inlet-outlet.

In a particular embodiment, the system comprises air injection and/or release means in connection with the interior of the cell separation chamber through the third fluid inlet-outlet. The injection of pressurized air into and/or the release thereof from the cell separation chamber advantageously contributes to the extraction of fluids from and the introduction thereof into said cell separation chamber.

In a particular embodiment, the flow control means for controlling the flow of fluid comprise
valves, preferably pinch valves; or
pumps; or
a combination of the above,
wherein the flow control means for controlling the flow of fluid are located in the duct network and are configured for regulating the passage of fluid.

In a particular embodiment, the means for driving fluids are connected to the duct network between the first inlet-outlet of the cell separation chamber and the residue receptacle.

In a particular embodiment, the system comprises at least one sensor connected to the duct network, wherein the sensor is preferably a bubble sensor.

In a particular embodiment, the system comprises a first sensor connected to the duct network between the cell separation chamber and the residue receptacle, and a second sensor connected to the duct network between the cell separation chamber and the reservoir.

A third inventive aspect provides a cell separation method for separating at least one cell system from its culture medium, the method being characterized in that it is implemented in the cell separation system according to the second inventive aspect, and in that it comprises the following steps:
a) filtering the content of the cell separation chamber through the plurality of microparticles and the first filter extracting the culture medium from the interior of the cell separation chamber through the first fluid inlet-outlet using the means for driving fluids in a first flow direction,
b) introducing a cleaning liquid or recovery medium into the interior of the cell separation chamber,
c) extracting the cleaning liquid or recovery medium through the first fluid inlet-outlet using the means for driving fluids in the first flow direction,
d) introducing a recovery medium into the interior of the cell separation chamber through the first fluid inlet-outlet using the means for driving fluids in a second flow direction,
e) extracting the cell system suspended in the recovery medium through the second fluid inlet-outlet going through the second filter in the second flow direction.

The present cell separation method advantageously allows completely separating the cultured cell system from the original culture medium (where said cell system was cultured).

In step a), the content of the cell separation chamber is understood to be a culture medium, a cell system and microparticles.

In a particular embodiment, when the cultured cell system is suspended in the culture medium in the interior of the cell separation chamber, the means for driving fluids are activated to thereby extract the culture medium from the interior of the cell separation chamber, filtering it between the microparticles that gradually retain the cell system, and subsequently filtering it through the first filter. In this step a) in which the culture medium is extracted, this culture medium is conveyed or circulated to the product receptacle in a first flow direction through the duct network. In a particular embodiment, in step a) the extraction of the culture medium is performed through the third fluid inlet-outlet of the cell separation chamber.

Once all or almost all the culture medium has been filtered, a cleaning liquid or recovery medium is introduced into the interior of the cell separation chamber (step b). In particular, the cleaning liquid or recovery medium is introduced in the first flow direction. After filling the cell separation chamber with the cleaning liquid or recovery medium, the means for driving fluids are activated to start the extraction of the cleaning liquid or recovery medium, entraining leftover culture medium which may remain in the interior of the cell separation chamber along with it. In this extraction, the liquid medium with those possible traces or leftover culture medium is filtered between the microparticles, retaining the cell system, and the first filter. In this step c) in which the cleaning liquid or recovery medium is extracted and leftover culture medium may also be extracted, this cleaning liquid or recovery medium or leftover culture medium are conveyed or circulated to the product receptacle, preferably to the residual product receptacles in a first flow direction through the duct network.

After step c), a recovery medium is introduced into the interior of the cell separation chamber in a second flow direction for the purpose of recovering the cultured cells. By introducing this recovery medium in a second flow direction, the interstices of the microparticles disappear and the cell system is no longer being retained. As the recovery medium is being introduced in step d) by activating the means for driving fluids in a second flow direction, the recovery medium with the cell system is gradually extracted from the interior of the cell separation chamber, filtering it through the second filter such that the microparticles are retained in the interior of the cell separation chamber. In this step e) in which the recovery medium and the cell system are extracted, said recovery medium and cell system are conveyed or circulated to the reservoir in the second flow direction through the duct network.

When steps a) and c) are carried out, the means for changing flow direction are operated so that the flow of the fluid is circulated in a first flow direction. However, when steps d) and e) are carried out, the means for changing flow direction are operated so that the flow of the fluid is circulated in a second flow direction, opposite the first flow direction. This first flow direction will be understood as the flow direction of the fluid when it goes from the interior of the cell separation chamber to the first fluid inlet-outlet, whereas the second flow direction will be understood as the flow direction of the fluid when it goes from the interior of the cell separation chamber to the second fluid inlet-outlet.

In a particular embodiment, before step a) the method comprises introducing at least one cell system suspended in a culture medium into the interior of the cell separation chamber.

Advantageously, the method allows being able to introduce at least one cell system suspended in a culture medium into the interior of the cell separation chamber before step a), regardless of whether or not there was cell system content already suspended in the culture medium beforehand.

In a particular embodiment, the cell separation method comprises repeating steps b) and c). Advantageously, it allows removing any minimal trace of culture medium or leftover culture medium that may remain in the interior of the cell separation chamber.

In a particular embodiment, the cell separation method comprises between steps d) and e) filtering the recovery medium (acting as a cleaning liquid) through the microparticles and the first filter, extracting said recovery medium from the interior of the cell separation chamber through the first fluid inlet-outlet using the means for driving fluids in the first flow direction, and subsequently repeating step d).

In another particular embodiment, the cell separation method comprises introducing a recovery medium into the interior of the cell separation chamber in a second flow direction and subsequently filtering said recovery medium through the microparticles and the first filter. In another particular embodiment, the cell separation method comprises introducing a recovery medium into the interior of the cell separation chamber through the second or fourth fluid inlet-outlet in a first direction so that said recovery medium is subsequently filtered through the microparticles and the first filter.

All the features and/or steps of methods described in this specification (including the claims, description and drawings) can be combined in any combination, with the exception of combinations of such mutually exclusive features.

### Description of the Drawings

These and other features and advantages of the invention will be more clearly understood based on the following detailed description of a preferred embodiment given only by way of illustrative and non-limiting example in reference to the attached drawings.
Figure 1 shows a schematic depiction of a cell separation chamber according to a particular embodiment of the present invention.
Figure 2 shows a schematic depiction of a cell separation system according to a particular embodiment of the present invention.
Figure 3 shows a schematic depiction of a cell separation system according to another particular embodiment of the present invention.
Figures 4A-4C show schematic depictions of three different states of the cell separation chamber according to a particular embodiment of the present invention.

### Detailed Description of the Invention

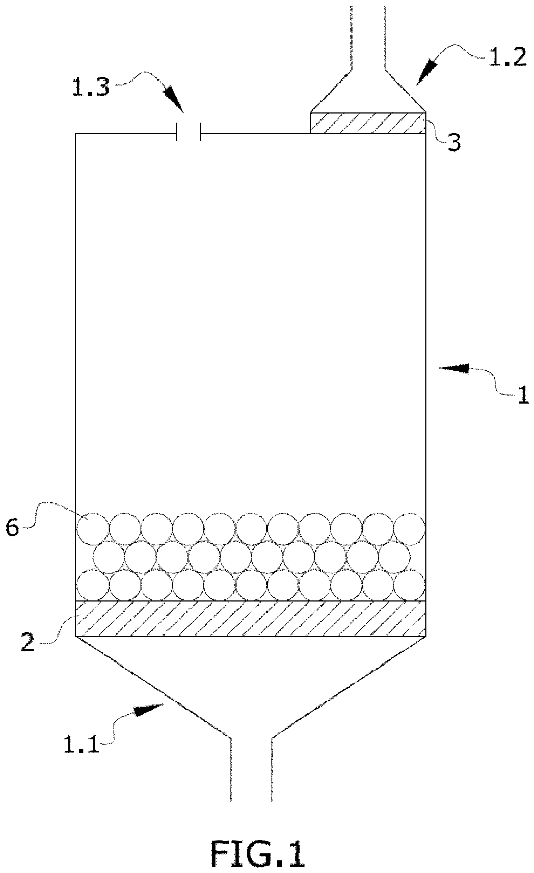

Figure 1 shows a particular example of the cell separation chamber (1) in the interior of which there is a plurality of microparticles (6) accumulated in the lower part thereof. The cell separation chamber (1) has a first fluid inlet-outlet (1.1) in which a first filter (2) is arranged, and a second fluid inlet-outlet (1.2) in which a second filter (3) is arranged. In this Figure 1, the microparticles (6) are depicted with a spherical geometry; however, these microparticles (6) may have any geometry. The main objective of these microparticles is that when a liquid medium is driven into the interior of the cell separation chamber (1) from the bottom to the top, i.e., from the first fluid inlet-outlet (1.1) to the interior of said chamber of cell recovery (1), the microparticles (6) are entrained by the flow of the entering fluid and separated from one another, thereby undoing the accumulation of said microparticles (6).

Furthermore, in the upper part of the cell separation chamber (1) there is a third fluid inlet-outlet (1.3) configured for being in fluid connection with air injection and/or release means (14) of the cell separation system through a first duct (7.1) (not shown in the drawing).

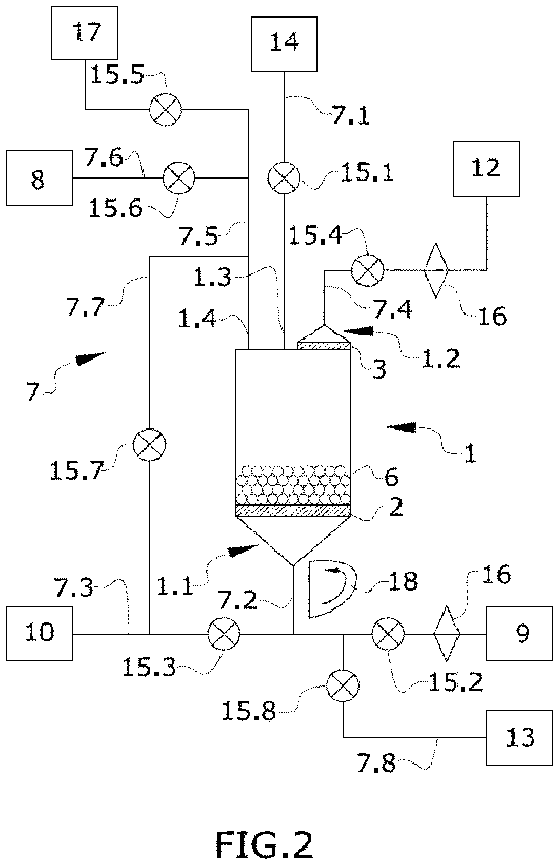

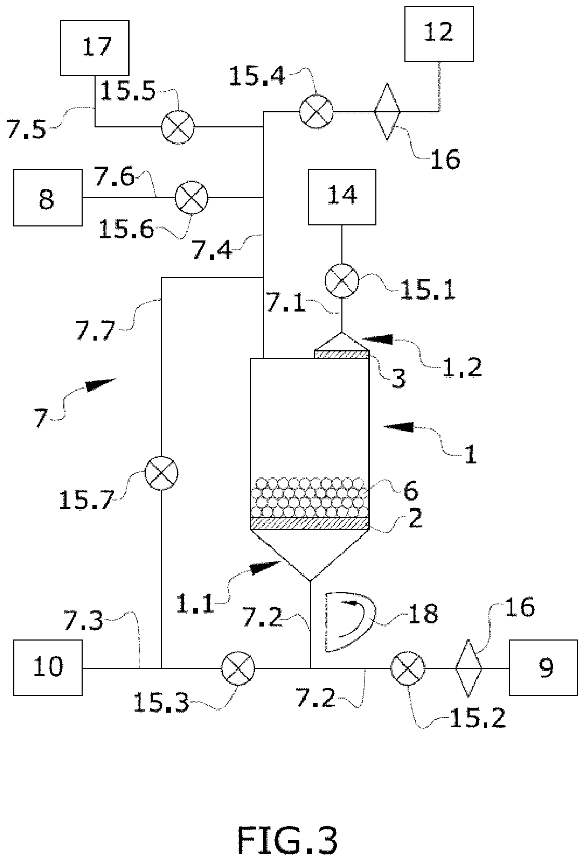

Figure 1 furthermore schematically shows how the first fluid inlet-outlet (1.1) and second fluid inlet-outlet (1.2) have a truncated cone shape comprising two openings on each base thereof, i.e., a larger opening corresponding with the larger base of said truncated cone, where the filters (2, 3) are arranged in direct contact with the interior of the cell separation chamber (1), and a smaller opening corresponding with the smaller base of the truncated cone, wherein each inlet-outlet (1.1, 1.2) connects with the duct network, particularly with ducts (7.2, 7.4), respectively, as shown in Figures 2 and 3.

Figures 2 and 3 show two particular embodiments of the cell separation system of the present invention. In these particular examples, the cell system to be separated from the culture medium is cells.

Figures 2 and 3 furthermore show the cell separation chamber (1), in the interior of which there is a plurality of microparticles (6) accumulated in the lower part thereof. The cell separation chamber (1) has a first fluid inlet-outlet (1.1) in which a first filter (2) is arranged, and a second fluid inlet-outlet (1.2) in which a second filter (3) is arranged. In a more particular example, said microparticles (6) were introduced into the cell separation chamber (1) from the upper part of this cell separation chamber (1), and due to the effect of gravity (from the upper part of the cell separation chamber to the lower part thereof), the microparticles (6) gradually accumulated in the lower part of said cell separation chamber (1) .

In both Figures 2 and 3, the first filter (2) is arranged along the entire larger opening of the truncated cone-shaped inlet-outlet (1.1), such that the first fluid inlet-outlet (1.1) is closed by said first filter (2). The second filter (3) is arranged along the entire larger opening of the truncated cone-shaped inlet-outlet (1.2), such that the second fluid inlet-outlet (1.2) is closed by the second filter (3).

In another particular example (not shown in the drawings), the first fluid inlet-outlet (1.1) and the second fluid inlet-outlet (1.2) of the cell separation chamber (1) have the same geometry as the third fluid inlet-outlet (1.3), such that the filters (2, 3) are arranged in the interior of these inlets-outlets (1.1, 1.2). So when a fluid is introduced into the cell separation chamber (1), for example through the first inlet (1.1), this fluid must go through the first filter (2) in order to be able to enter the interior of the cell separation chamber (1). Likewise, when a fluid is extracted from the cell separation chamber (1), for example through the second inlet-outlet (1.2), this fluid goes through the second filter (3) in order to be able to be extracted from the interior of the chamber.

Furthermore, in the upper part of the cell separation chamber (1) there is a third fluid inlet-outlet (1.3) in fluid connection with air injection and/or release means (14) through the duct network (7), particularly through a first duct (7.1). A first valve (15.1) (connected to the first duct (7.1)) is located between the air injection and/or release means (14) and the third fluid inlet-outlet (1.3). This first valve (15.1) is in charge of regulating the passage and release of air into/from the interior of the cell separation chamber (1).

The cell separation system shown in both Figures 2 and 3 furthermore has a residual product receptacle (9) that can be in fluid communication with the interior of the cell separation chamber (1) by means of the duct network (7) through the first fluid inlet-outlet (1.1), particularly by means of a second duct (7.2). This second duct (7.2) connects the residue receptacle (9) with the first fluid inlet-outlet (1.1) of the cell separation chamber (1). Furthermore, there is a second valve (15.2) connected to the second duct (7.2) in charge of regulating the passage of fluids through said second duct (7.2). For example, when filtration of a liquid medium (culture medium and/or cleaning liquid or recovery medium) from the interior of the cell separation chamber (1) through the first filter (2) is carried out, the second valve (15.2) controls the passage of said filtered liquid medium coming from the interior of the cell separation chamber (1) to the residue receptacle (9). In other words, the entire content of the interior of the cell separation chamber (1) that is considered negligible is circulated to the residual product receptacle (9).

In the particular example of Figure 2, the cell separation system further comprises an intermediate product receptacle (13) suitable for storing therein cell product and/or culture medium (4). In other words, if the culture medium (4) coming from the interior of the cell separation chamber was of interest, this culture medium (4) is collected in an intermediate product reservoir (13) for subsequent use. In other words, when the culture medium (4) in which the cells were cultured is a product of interest, said product can be collected in the system. The intermediate product receptacle (13) is connected to the second duct (7.2) to thereby be in fluid communication with the interior of the cell separation chamber (1) through an eighth duct (7.8). Furthermore, an eighth valve (15.8) in charge of regulating the passage of the culture medium (4) (not shown in the drawing) or cell product coming from the interior of the cell separation chamber (1) is located in this eighth duct (7.8) .

In a particular example, the cell separation system is configured for being in fluid communication with a bioreactor. In particular, when the cells (5) are cultured in a culture medium (4), all of which is contained in a bioreactor or bioreaction chamber, this content can be introduced into the present cell separation chamber (1). Once said content has been introduced into the present cell separation chamber, it is filtered such that the culture medium is extracted through the first inlet-outlet (1.1) to the intermediate product receptacle (13). The remaining content in the interior of the cell separation chamber which corresponds to cells are thereafter conveyed back into the bioreactor or bioreaction chamber.

The cell separation system shown in Figures 2 and 3 further comprises a recovery medium receptacle (10) that can be in fluid communication with the interior of the cell separation chamber (1) by means of the duct network (7), particularly by means of a third duct (7.3). The recovery medium receptacle (10) is connected with the first fluid inlet-outlet (1.1) of the cell separation chamber (1) through the second duct (7.2). In other words, the third duct (7.3) is connected to the second duct (7.2) between the first fluid inlet-outlet (1.1) and the second valve (15.2). Furthermore, a third valve (15.3) located in the third duct (7.3) is in charge of regulating the passage of the recovery medium (11) (not shown in the drawing) through said third duct (7.3). For example, once all the culture medium (4) that was in the interior of the cell separation chamber (1) has been extracted (by means of filtration), a recovery medium (11) is introduced into said cell separation chamber (1), this recovery medium (11) coming from the recovery medium receptacle (10) .

The cell recovery system shown in Figures 2 and 3 further comprises means for driving fluids, which in these examples are an alternating flow pump (18) located in the lower part of the cell separation chamber (1), connected to the duct network (7), particularly connected to the second duct (7.2) close to the first fluid inlet-outlet (1.1). The alternating flow pump (18) is in charge of circulating or driving the content of the cell separation chamber (1) as well as the fluids entering same. Furthermore, in these examples, the means for changing flow direction of the fluids are the alternating flow pump (18) itself which, in addition to driving the fluid, is capable doing so in the direction of interest. For example, during filtration of a liquid medium through the first filter (2), the alternating flow pump (18) is in charge of pumping the liquid medium being filtered from the interior of the cell separation chamber (1) to the second duct (7.2) in a first direction, which is understood as a downward direction, i.e., going towards the residue receptacle (9). In another example, during recovery of the cultured cells (5), the recovery medium (11) coming from the recovery medium receptacle (10) is pumped in a second direction, which is understood as an upward direction, i.e., from the first inlet-outlet (1.1) to the interior of the cell separation chamber (1). The upward direction provided by the alternating flow pump (18) is the direction the flow of fluid takes when it is introduced into the cell separation chamber (1) through the first fluid inlet-outlet (1.1), whereas the downward direction, opposite the upward direction, will be the direction the flow of fluid takes when it is released from the interior of the cell separation chamber (1) through the first fluid inlet-outlet (1.1) .

The cell recovery system shown in both Figures 2 and 3 furthermore has an end product receptacle (12), i.e., a cell recovery reservoir, in charge of storing therein recovered cells (4) suspended in the recovery medium (11), all coming from the interior of the cell separation chamber (1). This end product receptacle (12) can be in fluid communication with the interior of the cell separation chamber (1) through the second fluid inlet-outlet (1.2) by means of the duct network (7), particularly by means of a fourth duct (7.4). For example, in the cell recovery step, with the alternating flow pump (18) acting in an upward direction, the cultured cells (5) suspended in the recovery medium (11) are pumped to the upper part of the cell separation chamber (1) such that they are filtered through the second filter (3) where they are delivered to the end product receptacle (12). A fourth valve (15.4) connected to the fourth duct (7.4) is located between the end product receptacle (12) and the second fluid inlet-outlet (1.2). This fourth valve (15.4) is in charge of controlling the passage of cells (5) suspended in the recovery medium (11) coming from the interior of the cell separation chamber (1).

The present cell recovery system comprises bubble sensors (16) arranged in the duct network (7), particularly in the second duct (7.2) and fourth duct (7.4). These bubble sensors (16) are in charge of detecting/determining the time when fluid stops circulating through that section of the duct network where the bubble sensor (16) is located, which time corresponds with the passage of air instead of fluid. Furthermore, Figures 2 and 3 show how the system comprises a cell separation prechamber (17) where the cells (5) suspended in the culture medium (4) are initially stored, and a cleaning receptacle (8) where the cleaning liquid or PBS is stored.

In the particular example of Figure 2, the cell separation prechamber (17) and the cleaning receptacle (8) can be in fluid communication with the interior of the cell separation chamber (1) through a fourth fluid inlet-outlet (1.4) by means of the duct network (7). In particular, the cell separation prechamber (17) is connected to the fourth fluid inlet-outlet (1.4) by means of a fifth duct (7.5), whereas the cleaning receptacle (8) is connected to the fifth duct (7.5) through a sixth duct (7.6). Furthermore, in this particular example, between the cell separation prechamber (17) and the fourth fluid inlet-outlet (1.4) there is a fifth valve (15.5) connected to the fifth duct (7.5) which is in charge of controlling the passage of cells (5) suspended in the culture medium (4) coming from said cell separation prechamber (17) to the interior of the cell separation chamber (1). Furthermore, between the residue receptacle (8) and where the sixth duct (7.6) connects with the fifth duct (7.5), there is a sixth valve (15.6) in charge of controlling the passage of the cleaning liquid from the cleaning receptacle (8) to the interior of the cell separation chamber (1) .

In the particular example of Figure 3, the cell separation prechamber (17) and the cleaning receptacle (8) can be in fluid communication with the interior of the cell separation chamber (1) through the second fluid inlet-outlet (1.2) by means of the duct network (7). In particular, the cell separation prechamber (17) is connected to the second fluid inlet-outlet (1.2) through the fourth duct (7.4) by means of a fifth duct (7.5), whereas the cleaning receptacle (8) is connected to the fourth duct (7.4) through a sixth duct (7.6). Furthermore, in this particular example, between the cell separation prechamber (17) and where the fifth duct (7.5) connects with the fourth duct (7.4), there is a fifth valve (15.5) connected to the fifth duct (7.5) which is in charge of controlling the passage of cells (5) suspended in the culture medium (4) coming from said cell separation prechamber (17) to the interior of the cell separation chamber (1). Furthermore, between the residue receptacle (8) and where the sixth duct (7.6) connects with the fourth duct (7.4), there is a sixth valve (15.6) in charge of controlling the passage of the cleaning liquid coming from the cleaning receptacle (8) to the interior of the cell separation chamber (1). It can be seen in this particular example of Figure 3 how there is only one inlet-outlet, i.e., the second inlet-outlet (1.2), for both introducing the cells (5) suspended in the culture medium (4) into the interior of the cell separation chamber (1) and filtering and extracting the cells (5) suspended in the recovery medium (11).

Both Figures 2 and 3 show how the recovery medium receptacle (10) can be in fluid communication with the interior of the cell separation chamber (1) from the upper part thereof, i.e., through the fourth fluid inlet-outlet (1.4) (according to the example of Figure 2), or through the second fluid inlet-outlet (1.2) (according to the example of Figure 3). A seventh duct (7.7) connecting with the third duct (7.3) or with the fifth duct (7.5), and a seventh valve (15.7) located in said seventh duct (7.7) which is in charge of controlling the passage of the recovery medium (11) coming from the recovery medium receptacle (10) to the interior of the cell separation chamber (1) through the upper part thereof are shown in the present system.

The cell separation chamber (1) and the different reservoirs and receptacles can be in fluid communication with one another through a duct network (7) formed by a plurality of tubes or ducts (7.1, 7.2, 7.3, 7.4, 7.5, 7.6, 7.7). This duct network (7) comprises flow control means for controlling the flow of fluid in order to regulate the fluids leaving or entering the different receptacles or reservoirs and the cell separation chamber (1), respectively. These flow control means for controlling the flow of fluid are a series of pinch valves (15.1, 15.2, 15.3, 15.4, 15.5, 15.6, 15.7). Furthermore, the system comprises means for driving fluids that are understood as an alternating flow pump (18). In this particular example, this alternating flow pump (18) is also understood as means for changing flow direction.

### Cell separation method

The present cell separation system is in charge of carrying out the process of separating at least one cell system (5) from its culture medium (4), i.e., from the medium in which said cell system (5) were cultured in a preceding process. In this particular example, the cell system is cells. The cell separation method comprises the following steps:
a) filtering the content of the cell separation chamber (1) through the plurality of microparticles (6) and the first filter (2), extracting the culture medium (4) from the interior of the cell separation chamber (1) through the first fluid inlet-outlet (1.1) by means of the alternating flow pump (18) in a first direction (downward direction).

This first filtration step aims to completely extract the culture medium (4) from the interior of the cell separation chamber (1), such that the cells (5) remain together with the microparticles (6), forming a network, in the interior of the cell separation chamber (1) as a result of the combined function of the first filter (2) and the flow direction caused by the alternating flow pump (18).

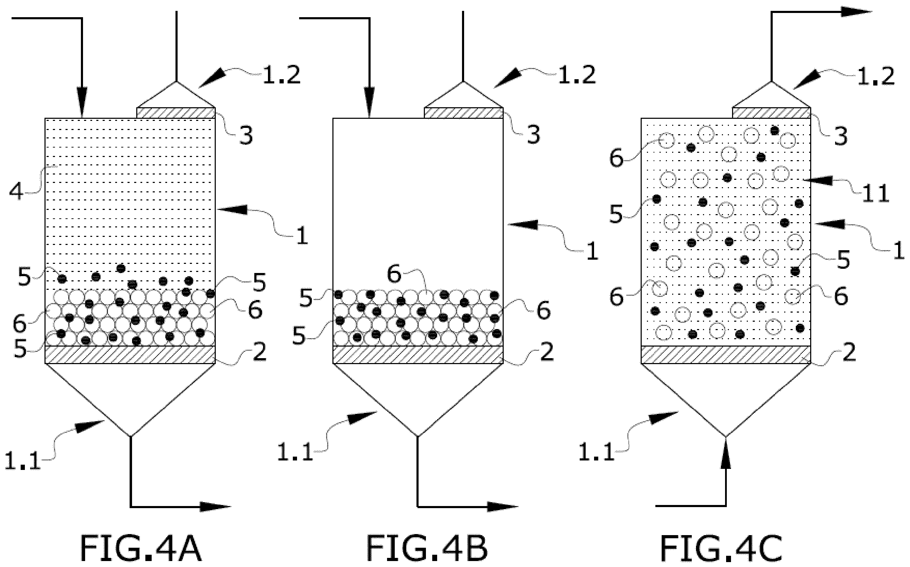

Figure 4A shows the state of the cell separation chamber (1) before starting the first filtration step a). It can be seen in particular how in the interior of the cell separation chamber (1) there are cells (5) and microparticles (6) accumulated in the form of a network in the lower part of the cell separation chamber and the culture medium (4) arranged between said cells (5) and microparticles (6). The arrows that are depicted in this Figure 4A show the first flow direction the fluid would have as soon as the first filtration step a) is started. This direction would correspond with the first flow direction.
b) introducing the cleaning liquid or recovery medium (11) into the interior of the cell separation chamber (1).

Once virtually all the culture medium (5) that was in the interior of the cell separation chamber (1) has been filtered, the system introduces a cleaning liquid or recovery medium into the cell separation chamber (1).
c) extracting the cleaning liquid or recovery medium through the first fluid inlet-outlet (1.1) by means of the alternating flow pump (18) in the first flow direction (downward direction).

This step c) aims to remove residual traces of culture medium (5) that may be present in the interstices of the network formed by the microparticles (6) and the cells (5). This is done by passing the cleaning liquid or PBS or recovery medium (11) coming from the cleaning receptacle (8) or the recovery medium receptacle (11), respectively, through the third fluid inlet-outlet (1.3). As it passes through the interior of the cell separation chamber, the cleaning liquid or PBS or recovery medium (11) entrains the residual traces of culture medium (5) and extracts them from the interior of the cell separation chamber (1) through the first fluid inlet-outlet (1.1) to the residual product receptacle (9).

Figure 4B shows the state of the cell separation chamber (1) after the second filtration step c). It can be seen in particular how there are cells (5) and microparticles (6) in the form of a network in the interior of the cell separation chamber (1). The arrows that are depicted in this Figure 4B show the first flow direction of the fluid during the second filtration step c).

Both in the first and second filtration steps a) and c), the pore size of the first filter (2) is large enough to allow the passage of the culture medium (5) and/or cleaning liquid or recovery medium, and small enough to prevent the passage of the microparticles (6) and cells contained in the interior of the cell separation chamber (1).
d) introducing a recovery medium (11) into the interior of the cell separation chamber (1) through the first fluid inlet-outlet (1.1) by means of the alternating flow pump (18) in a second flow direction (upward direction).

This step d) aims to introduce a recovery medium (11) with which cells (5) that have been separated from the culture medium (4) are recovered. The alternating flow pump (18) causes a change in flow direction in the interior of the cell separation chamber (1) when it introduces the recovery medium (11) through the first fluid inlet-outlet (1.1). Through the introduction of the recovery medium (11) in the upward direction, the network formed by the microparticles (6) and cells (5) is broken up.

Figure 4C shows the state of the cell separation chamber (1) in step d). It can be seen in particular how there are cells (5) and microparticles (6) suspended in the recovery medium in the interior of the cell separation chamber (1), i.e., once the network formed has broken up. The arrows that are depicted in this Figure 4C show the second flow direction the fluid would have in this step d) upon starting the extraction and recovery step e).
e) extracting the cells (5) suspended in the recovery medium (11) through the second fluid inlet-outlet (1.2) going through the second filter (3) in the second flow direction (upward direction).

Once the recovery medium (11) has been introduced into the interior of the cell separation chamber (1), and the network formed by the microparticles (6) and the cells has broken up, step e) for extracting/recovering the cultured cells (5) is started. In other words, a third filtration step is performed in which the cells (5) suspended in the recovery medium (11) are extracted through the second filter (3) for delivering them to the end product receptacle (12), the microparticles (6) being retained in the interior of the cell separation chamber (1). In this third filtration step e), the pore size of the second filter (3) is large enough to allow the passage of the recovery medium (11) with the cells (5) suspended therein, and small enough to prevent the passage of the microparticles (6) suspended in the recovery medium (11) in the interior of the cell separation chamber (1).

In a particular example, before the first filtration step a), the cells (5) suspended in the culture medium (4) are introduced into the interior of the cell separation chamber (1). These cells (5) suspended in the culture medium (4) in which they were cultured come from the cell separation prechamber (17) .

In a particular example, steps b) and c) can be repeated as many times as desired for the purpose of cleaning any trace of culture medium (4) that may remain in the interior of the cell separation chamber (1).

The functional scheme of the flow control means for controlling the flow of fluid in each of the steps according to the embodiment shown in Figure 2 is shown below:
Before step a), in the phase of introducing the cells (5) suspended in the culture medium (4), the functional scheme is as follows:
- the fifth valve (15.5) is open;
- the second and sixth valves (15.2 ,15.6) are closed;
- the first valve (15.1) is open;
- the alternating flow pump (18) is deactivated.

Once all the culture medium (4) and cells (5) suspended therein, coming from the cell separation prechamber (17), have been introduced into the interior of the cell separation chamber (1), the first filtration step in which the culture medium (4) is not of interest is started, the functional scheme in said step being as follows:
- the fifth valve (15.5) is closed;
- the first valve (15.1) is closed;
- the second valve (15.2) is open;
- the third and eighth valves (15.3, 15.8) are closed;
- the alternating flow pump (18) is activated, pumping in a first direction.

After the first filtration step a), for the purpose of cleaning from the interior of the cell separation chamber (1) any trace of culture medium (4) that may remain in the interstices between the microparticles (6) and cells (5), step b) of introducing the cleaning liquid or PBS into the interior of the cell separation chamber (1) is performed. The functional scheme in said step is as follows:
- the second valve (15.2) is closed;
- the sixth valve (15.6) is open;
- the first valve (15.1) is open;
- the alternating flow pump (18) is deactivated.

Once the cleaning liquid or PBS has been introduced into the interior of the cell separation chamber (1) during step b), step c) of extracting the liquid medium, cleaning liquid, is performed such that the operating scheme is as follows:
- the second valve (15.2) is open;
- the sixth valve (15.6) is closed;
- the first valve (15.1) is open;
- the alternating flow pump (18) is activated, pumping in the first direction

After steps b) and c) in which the interior of the cell separation chamber (1) was washed to remove the residual culture medium (4) with the cleaning liquid, step d) of introducing the recovery medium (11) into the interior of the cell separation chamber (1) for recovering the cells (5) is performed. The operating scheme in this step d) is as follows:
- the third valve (15.3) is open;
- the second valve (15.2) is closed;
- the first valve (15.1) is open;
- the fifth, sixth and seventh valves (15.5, 15.6, 15.7) are closed;
- the alternating flow pump (18) is activated, pumping in the second direction.

Step e) of extracting the recovery medium (11) for recovering the cells (5) suspended therein is then performed. In this step in which the cells (5) are recovered through the recovery medium (11), this mixture is stored in the end product receptacle (12), such that the functional scheme is as follows:
- the third valve (15.3) is open;
- the first valve (15.1) is closed;
- the fourth valve (15.4) is open;
- the alternating flow pump (18) is activated, pumping in the second direction.

Following the preceding sequence of steps that take place in the cell separation method, the cell separation chamber (1) of the cell separation system will be back in the initial state, i.e., free of traces of culture medium (4) and cells (5), having only the plurality of microparticles (6) therein. The chamber is therefore ready for the introduction of a new culture medium (4) containing new cells (5) cultured for their filtration and subsequent recovery.

## Claims

1. A cell separation chamber (1) suitable for storing fluids and/or cell systems (5) therein, the cell separation chamber (1) being **characterized in that** it comprises:
a first fluid inlet-outlet (1.1),
a second fluid inlet-outlet (1.2), and
filtering means comprising:
a first filter (2) arranged in the first fluid inlet-outlet (1.1),
a second filter (3) arranged in the second fluid inlet-outlet (1.2), and
a plurality of microparticles (6) arranged in the interior of the cell separation chamber (1),
wherein
the first filter (2) comprises a first plurality of pores, each pore comprising a first pore size which is smaller than the size of each component of the cell systems (5) and smaller than the size of the microparticles (6),
the second filter (3) comprises a second plurality of pores, each pore comprising a second pore size which is smaller than the size of the microparticles (6) and larger than the size of each component of the cell systems (5), and
the cell separation chamber (1) is configured for being in fluid communication with a duct network (7) of a cell separation system.

2. The cell separation chamber (1) according to claim 1, **characterized in that** the microparticles (6) occupy a volume between 10% and 50% of the interior of the cell separation chamber (1).

3. The cell separation chamber (1) according to any of the preceding claims, **characterized in that** the microparticles (6) are formed by polymeric materials, preferably biocompatible polymeric materials, such as polystyrene, polypropylene, polylactic acid, collagen, cellulose, chitosan, or any combination thereof.

4. The cell separation chamber (1) according to any of the preceding claims, **characterized in that** the first filter (2) and the second filter (3) have a pore size between 0.2 µm and 200 µm.

5. The cell separation chamber (1) according to any of the preceding claims, **characterized in that** the first filter (2) and the second filter (3) are formed by polymeric materials, preferably biocompatible polymeric materials, such as polycarbonate, or cellulose, or nylon, or PES, or PE, or PEEK, or PET, or any combination thereof.

6. The cell separation chamber (1) according to any of the preceding claims, **characterized in that** it comprises a third fluid inlet-outlet (1.3), said third fluid inlet-outlet (1.3) being configured so that the fluid is preferably air.

7. A cell separation system, comprising:
at least one cell separation chamber (1) according to any of claims 1 to 6,
a duct network (7) connected with the interior of the at least one cell separation chamber (1),
flow control means for controlling the flow of fluid circulating through the duct network (7),
means for driving fluids entering and leaving the at least one cell separation chamber (1),
at least one product receptacle connected to the duct network (7) and suitable for housing therein fluids coming from the interior of the cell separation chamber (1), and
a recovery medium receptacle (10) connected to the duct network (7) and storing a recovery medium (11) therein,
wherein the system is configured for changing the flow direction of the fluid entering and/or leaving the at least one cell separation chamber (7) using means for changing flow direction.

8. The system according to claim 7, **characterized in that** it comprises:
a residual product receptacle (9) connected to the duct network (7) and suitable for housing therein residual fluids coming from the interior of the cell separation chamber (1), and/or
an end product receptacle (12) connected to the duct network (7) and configured for housing therein fluids coming from the interior of the cell separation chamber (1), preferably cell systems (5) suspended in a recovery medium (11).

9. The system according to any of claims 7 to 8, **characterized in that** it further comprises a cleaning receptacle (8) connected to the duct network (7) and storing a cleaning liquid therein, this cleaning receptacle (8) being configured for releasing cleaning liquid into the interior of the cell separation chamber (1).

10. The system according to any of claims 7 to 9, **characterized in that** it comprises a cell separation prechamber (17) connected to the duct network (7), the cell separation prechamber being suitable for storing a cell system (5) suspended in a culture medium (4).

11. The system according to any of claims 7 to 10, **characterized in that** it comprises air injection and/or release means (14) in fluid connection with the interior of the cell separation chamber (1) through the third fluid inlet-outlet (1.3) according to claim 7.

12. The system according to any of claims 7 to 11, **characterized in that** the flow control means for controlling the flow of fluid comprise:
valves (15), preferably pinch valves, or
pumps, or
a combination of the above,
wherein the flow control means for controlling the flow of fluid are located in the duct network (7) and are configured for regulating the passage of fluid.

13. The system according to any of claims 7 to 12, **characterized in that** it comprises at least one sensor (16) connected to the duct network (7), wherein the sensor is preferably a bubble sensor.

14. A cell separation method for separating at least one cell system (5) from its culture medium (4), **characterized in that** it is implemented in the cell separation system according to claims 7 to 13, and **in that** it comprises the following steps:
a) filtering the content of the cell separation chamber (1) through the plurality of microparticles (6) and the first filter (2), extracting the culture medium (4) from the interior of the cell separation chamber (1) through the first fluid inlet-outlet (1.1) using the means for driving fluids in a first flow direction,
b) introducing a cleaning liquid or recovery medium (11) into the interior of the cell separation chamber (1),
c) extracting the cleaning liquid or recovery medium (11) through the first fluid inlet-outlet (1.1) using the means for driving fluids in the first flow direction,
d) introducing a recovery medium (11) into the interior of the cell separation chamber (1) through the first fluid inlet-outlet (1.1) using the means for driving fluids in a second flow direction which use means for changing flow direction,
e) extracting the cell system (5) suspended in the recovery medium (11) through the second fluid inlet-outlet (1.2) going through the second filter (3) in the second flow direction.

15. The method according to claim 14, **characterized in that** it comprises, before step a), introducing at least one cell system (5) suspended in the culture medium (4) into the interior of the cell separation chamber (1).
